# EUROPEAN PATENT SPECIFICATION

(11) **EP 0 403 015 B1**
(45) Date of publication and mention of the grant of the patent: **07.12.1994**
(21) Application number: 90201504.9
(22) Date of filing: 12.06.1990
(51) Int. Cl.: C12N 5/00, C12M 3/04

(54) **Method for culturing cells**
Zellzüchtungsverfahren
Méthode pour cultiver des cellules

(30) Priority: 16.06.1989 NL 8901520
(43) Date of publication of application: 19.12.1990
(73) Proprietor: Akzo Nobel N.V., 6824 BM Arnhem (NL)
(72) Inventor: Schönherr, Otto Thomas, NL-5344 EB Oss (NL); Sanders, Adrianus Lambertus Maria, NL-5406 PP Uden (NL)
(74) Representative: Hermans, Franciscus G.M.

(56) References cited:
- EP-A- 0 021 257
- EP-A- 0 183 207
- EP-A- 0 310 911
- GB-A- 2 203 447
- PATENT ABSTRACTS OF JAPAN, vol. 11, no. 80 (C-409)[2527], 11th March 1987, page 43 C 409

## Description

The invention relates to a method for culturing cells, which are incubated in a suitable culture medium, and to the use of cylindrical sheaths in such method.

The culture of cells on an industrial scale has already been used for centuries in, inter alia, foodstuffs technology. However, the cells concerned in this case were mainly microorganisms, such as bacteria and yeasts, which tolerate fairly large variations in culture conditions.

The culture of more sensitive cell types, such as mammalian cells, which, for example, produce substances such as cytokines, plasminogen activators or monoclonal antibodies which can be used for medical purposes, is of more recent date. The greater sensitivity of these cell types is frequently due, inter alia, to the absence of a strong cell wall (which in the case of bacteria and yeast surrounds the plasma membrane) and the lower tolerances under process conditions.

The cell types without a cell wall are more sensitive to mechanical forces, which, for example, arise when stirring these cells in the culture vessel and when passing (oxygen-rich) gas mixtures through the culture.

Moreover, many mammalian cells grow only when they are attached to a surface.

Various culture methods have been developed for culturing such sensitive cell types on an industrial scale, which methods solve some of the problems but also have their disadvantages. Thus, cells of this type are cultured, for example, with the aid of hollow fibres or on microscopically small spheres (micro-carriers).

In the case of culture systems using bundles of hollow fibres, a medium which contains nutrients flows through the many hundreds of fairly long, semi-permeable tubes and the cells grow on the outside of these tubes. Depending on their molecular dimensions in relation to the pore size of the membranes, the products which these cells secrete will diffuse back to the lumen of the tubes and/or accumulate in the fluid which surrounds the cells.

Scaling up culture systems of this type is technologically difficult.

The culture of cells on microscopically small spheres is attractive because use can be made of the same types of culture vessels which have traditionally also been used for bacteria, yeasts, etc. and have been extensively optimized with respect to the provision with nutrients. Nevertheless, with this culture method the vulnerability of the cells still constitutes a limitation. The cells are located on the surface of the small spheres and will be damaged if the mechanical forces which arise during vigorous stirring and forced aeration by passing gas bubbles through the culture are too great. Many mammalian cells have the tendency to grow out three-dimensionally on micro-carries of this type - several cell layers form on the surface of the sphere - and lumps of cells or loose cells detach from the surface of the sphere in the course of time. In addition, with perfusion cultures on a large scale the streams of fluid to be removed (which may contain the desired product) are large, while the settling rate of the micro-carriers is generally low. As a result, micro-carriers of this type are difficult to separate off by settling.

A culture method has now been found with which these drawbacks do not arise.

This method is characterized in that the cells are located in a virtually cylindrical sheath (preferably a capillary) which over the entire length has been brought into contact with the culture medium and the hollow interior cavity of which is also in open contact with the culture medium via at least one of the two ends of the cylindrical sheath; the sheaths may, however, be open at both sides as well.

In general, cylindrical sheaths with a length:diameter ratio of approximately 1:2 to 100:1, and preferably 1:1 to 25:1, are used in this method.

The sheaths can, for example, have a diameter of approximately 0.01-1 mm, and for use with mammalian cells preferably have a diameter of 0.1-1 mm.

In order to achieve good culture results with surface-dependent cells it is important that the cells adhere to the small cylinders. Depending on the composition of the material from which the sheaths are produced, it can be necessary to apply a coating, for example of collagen or of gelatin, to the carrier material, or chemically to modify the carrier material, in which case, for example, positively or negatively charged groups are introduced.

With the culture method according to the invention both stirred and non-stirred culture vessels can be used. These options provide various degrees of freedom with regard to the nature and characteristics of the material from which the sheaths can be produced. In the case of non-stirred culture conditions, less stringent demands are made with respect to the ruggedness and robustness of the sheaths, and also to the wall thicknesses thereof. Moreover, the specific weight of the sheaths can vary. In both fixed bed, fluidized bed and stirred systems the preference is for particles which are relatively heavier than the micro-carriers which are now customary. In fluidized bed systems higher fluid circulation speeds and higher reactors can be used as a result of heavier particles.

In stirred systems, heavier small cylinders offer advantages in the separation thereof. The forces which are generated by the higher stirring speed which is needed to keep the small cylinders in suspension but which is also advantageous for an optimum oxygen supply has now adverse effect on the cells within their cylindrical sheath.

The sheaths according to the invention can be produced from diverse types of materials. It can be practical in use, but it is not absolutely essential, that the material is transparent. Simple visual observation is then frequently already sufficient for the experienced user to make an adequate diagnosis in the event of problems.

The material of the sheaths may or may not be permeable. The materials are in general polymers of synthetic or biological origin which may or may not be chemically modified and/or coated. Suitable non-permeable materials are, for example, glass and plastics (such as nylon and polypropylene). Suitable permeable sheath materials are, for example:
- semi-permeable membrane materials, such as those which are customarily used as dialysis membranes and ultra filtration membranes (for example cellulose);
- porous materials, such as those which are customarily used for microfiltration. These can be prepared by netting, or are rendered microporous by physical methods;
- gas-permeable materials (for example polysilanes);
- macrofilter materials, which thus have larger pores, provided these are smaller than the cell dimensions;
- biodegradable polymer materials (for example polylactic acid).

The sheaths according to the invention can, for example, be made by dividing long capillary fibres into pieces of suitable length.

The method according to the invention has a number of unexpected advantages which make this method suitable for various types of cells. The latter can be normal cells, cancer cells or cells modified with the aid of genetic manipulation. In the first place, the present method is suitable for surface-dependent cells, such as many types of eucaryote cells. In addition this method is also very suitable for cells which do not necessarily have to adhere, such as certain mammalian cells and plant cells which, in a protected environment (for example sheath), frequently grow into colonies or cell aggregates. It can also advantageously be used with aggregating single-cell organisms, such as moulds and, for example, blood cells and hybrid cells derived therefrom.

The method according to the invention is particularly suitable for the culture of cells which produce, and preferably secrete, specific useful substances. In addition, this method can also be used to produce a cell mass, which, for example, is suitable as inoculation material for purification of waste streams and as artificial seed.

For use in the method according to the invention, the desired cells can, for example, first be inoculated into long capillary fibres, and these fibres are then divided into short pieces of suitable length, which are then transferred to the culture medium. An alternative is to inoculate the desired cells into a culture vessel in which the cylindrical sheaths according to the invention are located.

In addition to the solid micro-carriers already mentioned above, macroporous micro-carriers are also sometimes used in cell culture; it is true that these have the advantage over solid micro-carriers that some of the cells can grow in a more or less protected environment within the macropores, but nevertheless they have many disadvantages compared with the small hollow cylinders according to the invention;
- in the case of the hollow cylinders the opening for cell-ingrowth is defined and constant, while the macroporous carriers have openings all around which are not defined and constant;
- the small hollow cylinders can be made of completely transparent material so that cell growth can readily be followed visually, while in the conventional macroporous carriers the individual cells are very difficult to detect;
- where small hollow cylinders with permeable walls are used, the content of these cylinders can, in principle, be increased without limitation by increasing the length; on increasing the dimensions of the micro-carriers (which after all are spherical) diffusion limitation for oxygen and nutrients and waste substances arises;
- micro-carriers (both solid and macroporous) display bridge-forming during use in cell cultures, as a result of which particles aggregate and an inhomo geneous suspension is formed; small hollow cylinders do not show this disadvantage.

### Example

### Culture of CHO.FSH cells on various types of micro-carriers

The experiment described here demonstrated that the influence of sheer stresses on cells cultured in cylindrical sheaths according to the invention is much less than in the case of other micro-carriers which are already known (Cytodex 3 (Pharmacia) and Cultispher G (Percell)).

For this experiment CHO cells were used which have been genetically modified by the use of recombinant DNA methods in such a way that they are capable of producing follicle-stimulating hormone (FSH).

The cylindrical sheaths which were used in this experiment are on average approximately 500 µm long, have a diameter of 200 µm and a wall thickness of 11 µm (dimensions in the dry state).

The cell culture took place in silicone-coated roller bottles. Ham's F₁₂/DMEM medium containing 5% FSC (fetal calf serum) was used during the inoculation and the growth of the cells.

As soon as the micro-carrier surfaces were completely grown over, culturing was continued with serum-free medium. The same amount of product was formed per cell in the various cultures.

In the cultures with Cytodex 3 and Cultispher G micro-carriers there was a relatively large number of cell aggregates and bridge formation occurred via the cells between different micro-carriers.

In the culture with cellulose cylinders there was no bridge formation and far fewer cell aggregates.

The settling rates of the various micro-carriers are given in Table 1 below.

**Table 1**

| Micro-carrier | Settling rate cm/min |
|---|---|
| Cytodex 3 | 1.3 |
| Cultispher G | 1.7 |
| Cellulose cylinders | 28 |

A 5 ml sample was taken from the roller bottle cultures and placed in a 10 ml test tube, which is then shaken vigorously for 6 minutes with the aid of a vortex mixer at 2,500 rpm.

The influence of shaking on the cell adhesion is measured by determining the increase in the quantity of loose cells in the supernatant. The results are given in Table 2.

**Table 2**

| Influence of vigorous shaking on the adhesion of cells to or in various micro-carriers. | | |
|---|---|---|
| Micro-carrier | Number of cells in supernatant^{a} | |
| | before shaking | after shaking |
| Cytodex 3 | 8% | 85% |
| Cultispher G | 3% | 60% |
| Cellulose cylinders | 2% | 8% |

| | | |
|---|---|---|
| ^{a} Numbers of cells are expressed in percentages of the numbers of cells present on or in the micro-carriers before shaking. | | |

Higher cell concentrations can be achieved with the cellulose cylinders. The maximum Cultispher G concentration is 5 g/l and the maximum Cytodex 3 concentration is 25 g/l; above these limits a viscous suspension forms (gel formation).

Cellulose cylinders can be used up to 40 g/l without a viscous suspension forming.

The following conclusions can be drawn.
- The FSH production per cell is the same for all types of micro-carriers.
- There are far fewer loose cells and cell aggregates in cultures with cellulose cylinders than in cultures with conventional micro-carriers. Bridge formation does not occur between cellulose cylin ders but does occur between Cytodex or Cultispher micro-carriers.
- The settling rate of cellulose cylinders is higher than that of the other micro-carriers, which promotes the cell separation from the product stream.
- The cells in cellulose cylinders are much better protected against shear forces as a consequence of vigorous mixing than cells in Cultispher G or on Cytodex 3 micro-carriers.
- Higher concentrations of cellulose cylinders can be used than of Cultispher G or Cytodex 3. These higher con centrations will have no adverse influence on the sensi tivity of cells to shear forces.

## Claims

1. Method for culturing cells located in hollow, virtually cylindrical sheaths with a diameter of 0.01-1 mm and a length:diameter ratio of 1:2 to 100:1, which sheaths are suspended in a suitable culture medium in a culture vessel whereby the hollow cavities of said sheaths are in open contact with the culture medium via at least one of the two ends, characterised in that the culture medium is stirred.

2. Method according to claim 1, characterised in that the length:diameter ratio of the cylindrical sheath is 1:1 to 25:1.

3. Method according to claim 1 or 2, characterized in that the cylindrical sheaths are made of cellulose.

4. Method according to any of claims 1-3, characterized in that cells are cultured which produce a desired product and this product is isolated from the cell culture.

5. Method according to claim 4, characterized in that the culturing and isolation of the product is a continuous process.

6. Use of hollow, virtually cylindrical sheaths, the hollow interior cavity of which is open at at least one of the two ends, which sheaths have a diameter of 0.01-1 mm and a length:diameter ratio of 1:2 to 100:1, to decrease the influence of the mechanical forces on cells in stirred cell cultures in a culture vessel wherein the cells are immobilized on said sheaths said sheaths are suspended in the medium.

## Patentansprüche

1. Verfahren zur Züchtung von Zellen, die sich in hohlen, nahezu zylindrischen Scheiden mit einem Durchmesser von 0,01-1 mm und einem Längen-:Durchmesserverhältnis von 1:2 bis 100:1 befinden, welche Scheiden in einem geeigneten Kulturmedium in einem Kulturgefäss suspendiert werden, wobei die Hohlräume besagter Scheiden in offenem Kontakt mit dem Kulturmedium über mindestens eines der beiden Enden stehen, dadurch gekennzeichnet, dass das Kulturmedium gerührt wird.

2. Verfahren gemäss Anspruch 1, dadurch gekennzeichnet, dass das Längen-:Durchmesserverhältnis der zylindrischen Scheide 1:1 bis 25:1 beträgt.

3. Verfahren gemäss Anspruch 1 oder 2, dadurch gekennzeichnet, dass die zylindrischen Scheiden aus Zellulose hergestellt werden.

4. Verfahren gemäss einem der Ansprüche 1-3, dadurch gekennzeichnet, dass Zellen gezüchtet werden, die ein erwünschtes Produkt herstellen und dieses Produkt aus der Zellkultur isoliert wird.

5. Verfahren gemäss Anspruch 4, dadurch gekennzeichnet, dass die Züchtung und Isolierung des Produkts ein fortlaufender Prozess ist.

6. Anwendung von hohlen, nahezu zylindrischen Scheiden, deren innerer Hohlraum mindestens an einem der beiden Enden offen ist, welche Scheiden einen Durchmesser von 0,01-1 mm und ein Längen-:Durchmesserverhältnis von 1:2 bis 100:1 haben, um den Einfluss mechanischer Kräfte auf Zellen in gerührten Zellkulturen in einem Kulturgefäss, worin die Zellen auf besagten Scheiden immobilisiert sind, zu erniedrigen, besagte Scheiden sind in dem Medium suspendiert.

## Revendications

1. Procédé pour la culture de cellules situées dans des gaines creuses virtuellement cylindriques ayant un diamètre de 0,01 à 1 mm et un rapport longueur/diamètre de 1/2 à 100/1, lesquelles gaines sont en suspension dans un milieu de culture convenable dans un récipient de culture, de telle sorte que les cavités creuses de ces gaines sont en contact ouvert avec le milieu de culture par l'intermédiaire d'au moins l'une des deux extrémités, caractérisé en ce que le milieu de culture est agité.

2. Procédé selon la revendication 1, caractérisé en ce que le rapport longueur/diamètre de la gaine cylindrique est de 1/1 à 25/1.

3. Procédé selon les revendications 1 ou 2, caractérisé en ce que les gaines cylindriques sont constituées de cellulose.

4. Procédé selon l'une quelconque des revendications 1 à 3, caractérisé en ce que l'on cultive des cellules qui produisent un produit désiré et que l'on isole ce produit à partir de la culture de cellules.

5. Procédé selon la revendication 4, caractérisé en ce que la culture et la séparation du produit constituent un procédé continu.

6. Utilisation de gaines creuses virtuellement cylindriques, dont la cavité intérieure creuse est ouverte au niveau d'au moins de l'une des deux extrémités, lesquelles gaines ont un diamètre de 0,01 à 1 mm et un rapport longueur/diamètre de 1/2 à 100/1, pour diminuer l'influence des forces mécaniques sur des cellules dans des cultures de cellules agitées dans un récipeint de culture, dans laquelle les cellules sont immobilisées sur ces gaines, ces gaines étant en suspension dans le milieu.
